(19) Europäisches Patentamt
European Patent Office
Office européen des brevets

(11) **EP 4 578 465 A1**

(12) **EUROPEAN PATENT APPLICATION**

(43) Date of publication:
**02.07.2025 Bulletin 2025/27**

(21) Application number: **23220542.7**

(22) Date of filing: **28.12.2023**

(51) International Patent Classification (IPC):
***A61K 49/00*** *(2006.01)* ***A61L 15/00*** *(2006.01)*

(52) Cooperative Patent Classification (CPC):
(C-Sets available)
**A61K 49/0043; A61K 49/0002; A61K 49/006; A61K 49/0073; A61K 49/0084; A61K 49/0089; A61L 15/56; A61L 15/60; C12Q 1/04; G01N 33/54313; G01N 33/569; G01N 33/582; G01N 33/84** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Paul Hartmann Aktiengesellschaft
89522 Heidenheim (DE)**

(72) Inventors:
- **KETTEL, Markus
89522 Heidenheim (DE)**
- **BAUER, Jochen
89522 Heidenheim (DE)**
- **BASTIDA AGOTE, Nora
89522 Heidenheim (DE)**

(74) Representative: **Ter Meer Steinmeister & Partner Patentanwälte mbB
Nymphenburger Straße 4
80335 München (DE)**

# (54) INFECTION DETECTION SYSTEM

(57) The invention relates to an infection detection system comprising a) a hydrogel, b) particles comprising liposome and first dye, c) particles comprising a carrier and pH indicator and/or particles comprising a pH-sensitive polymer and a second dye, d) particles comprising polymer having at least one carboxylic group and a third dye having a phenolic, aniline and/or anilide structure.

**Figure 9**

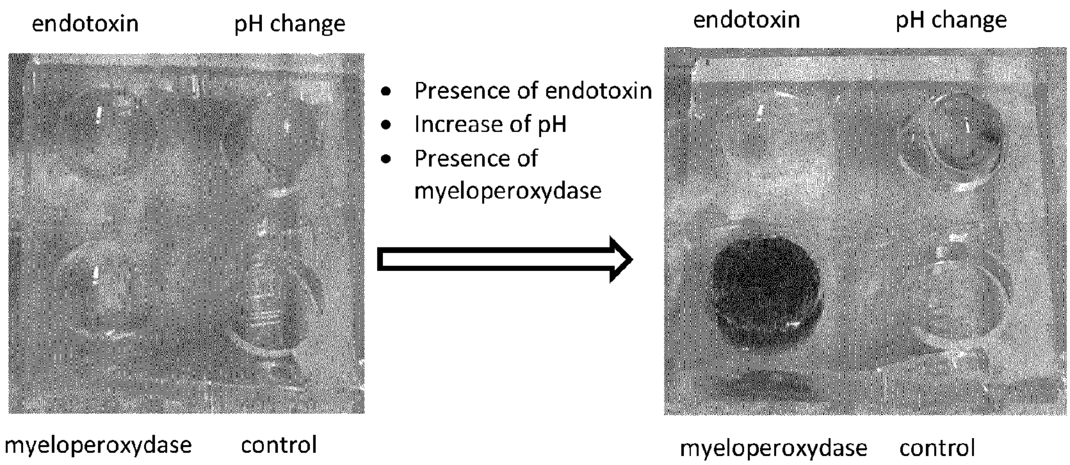


EP 4 578 465 A1

(52) Cooperative Patent Classification (CPC): (Cont.)

C-Sets
**A61L 15/60, C08L 75/04**

## Description

The invention relates to an infection detection system comprising a) a hydrogel, b) particles comprising liposome and first dye, c) particles comprising a carrier and pH indicator and/or particles comprising a pH-sensitive polymer and a second dye, d) particles comprising polymer having at least one carboxylic group and a third dye having a phenolic, aniline and/or anilide structure.

A wound can be regarded as the separation of the contiguity of tissues of the skin, wherein this is usually combined with a loss of substance.

The healing of wounds is based on the ability of the skin to regenerate tissue such as epithelial tissue, connective and supporting tissue. The regeneration of tissue is a complex occurrence of cell activities overlapping with each other, wherein said cell activities promote the healing process step by step. An accumulation of wound exudate, which might contain inter alia blood, proteins, residues of cells, microorganisms and leucocytes, can promote the growth of bacteria.

It is reported that the metabolism during wound healing is dependent on biochemical reactions which, when having properly taken place, ensure a good healing of the wound. For example, with reference to Shula VK, et al.: "Evaluation of pH measurement as a method of wound assessment", J. Wound Care, 2007; 16(7), pp. 291-294, a slightly acidic pH value of about 5.5 corresponding to the pH value of the outer surface of the skin is considered to promote oxygen perfusion, decreased bacteria presence, decreased proteolytic activity from proteases and enhanced fibroblast growth.

However, there are several unfavourable conditions which can slow down or even stop the process of wound healing. One of these unfavourable conditions is an infection. Generally, an infection can be referred to as the presence of replicating pathogenic microorganisms. These pathogenic microorganisms can produce waste products such as endotoxins and ammonia which can contribute to the elevation of the pH level of the wound to undesired alkaline levels. Another indication of an infection might be a host response, for example the presence of certain enzymes which for example can help to oxidize the undesired biomolecules such that the corresponding apoptotic material can be revealed and included by phagocytes.

In summary, though there is no unambiguous indication of an infection, there are several conditions which, when fulfilled, indicate an infection.

Taking into account the importance of timely handling a possible infection, the early and unambiguous detection of such an infection is of the utmost importance.

There are several approaches for infection detection systems. Some of them are based on the detection of one specific indication, for example a raised pH value, for an infection. However, said systems seem to be improvable since an indication of infection does not mandatorily mean that there is indeed an infection and handling a not-infected wound is not only time and cost-consuming but also might be negative with regard to the healing process, for example due to the trauma involved with a superfluous change of a wound dressing and the "infection" treatment itself. Thus, it seems to be recommendable to rely on more than just one indicator for determining whether there is an infection or not.

WO 2012/074509 A1 describes methods, devices and systems for patch-based physical, physiological, chemical and biochemical sensors that diagnose and monitor disease states. The patch-based sensors are to provide a panel of specific analyte parameters that determine one or more physiological conditions and/or the level of healing progression of a wound. However, said systems seem to be improvable with regard to reliability and the possibility of visual inspection.

Hence, it is an object of the present invention to provide an infection detection system which overcomes at least one of the shortcomings of the prior art.

Thus, there is still a need for an infection detection system with which a reliable detection of infections can be carried out. In particular, a system enabling a clear diagnosis of whether an infection is present or not should be provided. With regard thereto, it should be ensured that the presence of an infection is triggered by compounds derived from the wound and not by compounds of the infection detection system itself.

Moreover, a system accidentally indicating the presence of an infection, for example due to the presence of dirt or harmless bacteria from the surroundings, should be prevented.

Further, a system enabling the detection of an infection as early as possible should be provided such that the corresponding treatment can be timely applied, and the negative impacts of an infection can be reduced or even prevented. In other words, an infection detection system should be provided which helps to improve wound healing.

## Summary

The present invention has unexpectedly solved at least one of the above objectives by a new wound system comprising a hydrogel and three different kinds of particles, wherein each of said particles can be regarded as a detection system for one specific marker indicating the presence of an infection. The more detection systems for one specific marker indicate the presence of an infection, the more reliable said indication. In other words, a system based on a detection system for just one marker of an infection is prone to any kind of contamination such as dirt or to malfunction. A system able

to detect several markers substantially excludes the causal misfunction of the complete system even in case that one detection system for a specific single marker may not detect appropriately.

Thus, the subject of the present invention is an infection detection system comprising

a) hydrogel

b) particles comprising liposome and first dye,

c) particles comprising a carrier and pH indicator and/or particles comprising a pH-sensitive polymer and a second dye,

d) particles comprising polymer having at least one carboxylic group and a third dye having a phenolic, aniline and/or anilide structure.

A further subject of the present invention is a wound dressing comprising the infection detection system according to the present invention.

**Detailed Description**

The components a) to d) of the infection detection system according to the present invention are described in detail.

The infection detection system according to the present invention *inter alia* comprises a) a hydrogel.

In line with the present application a hydrogel is referred to as a synthetic or natural polymeric material, preferably a hydrophilic synthetic or natural polymeric material, which is capable of gel formation within a liquid, preferably water. The synthetic or natural polymeric material can be regarded as a matrix or hydrogel matrix.

The selected hydrogel comprised by the present composition can be preferably based on natural polymeric material. Non-limiting examples are starch, dextran, pectin, alginate, chitosan, hyaluronic acid, gellan, polypeptide and cellulose. Optionally, the natural polymeric material can be further processed, for example by chemical derivation, such as the formation of esters and ethers or pharmaceutically acceptable salts.

Starch is a polysaccharide containing a large number of $\alpha$-D-glucose units which are joined by glycosidic bonds. Starch can be produced by mot plants and used as energy storage. Starch is regarded as insoluble in cold water (23°C) but is able to exothermically swell up by physically binding a morefold of water compared to its own weight.

Dextran is a complex branched polysaccharide containing glucose units only, wherein the chains have different number average molecular weights from 10.000 to 50.000.000 Daltons determined by gel permeation chromatography. The straight chain consists of glucose molecules being bonded by $\alpha$-1,6 glycosidic linkages, while branches begin from $\alpha$-1,3 linkages. Brought into contact with water, dextran forms highly viscous liquids (gels).

Pectin is a vegetable structural polysaccharide obtainable from plants such as citrus fruits. Pectin is a polysaccharide (more exactly a polyuronide) which mainly contains D-galacturonic acid units being $\alpha$-1,6 glycosidicly joined. All, just a part or none of the carboxy residues of the galacturonic acid in pectin can be present in an esterified form, for example as methyl or ethyl ester.

Alginate is a polysaccharide containing homopolymeric blocks of (1-4)-linked $\beta$-D-mannuronate and $\alpha$-L-guluronate (G) residues, respectively, covalently linked together in different sequences or blocks. Alginate can be present in form of its salts, such as alkaline metal or earth alkaline metal salts, or in esterified form, such as in form of an alkyl ester, preferably methyl ester. Alginate is able to quickly absorb water to form a hydrogel. Thus, alginate can be used as gelling agent.

Chitosan is a linear biopolymer (polysaccharide) containing randomly distributed $\beta$-(1,4)-linked D-glucosamine units (deacetylated unit) and N-acetyl-D-glucosamine units, wherein the polymeric chain preferably contains more D-glucosamine units than N-acetyl-D-glucosamine units. Chitosan can be obtained from chitin by deacetylation either enzymatically or via an alkaline compound such as sodium hydroxide.

Hyaluronic acid is an important component of the connective tissue. Hyaluronic acid is a polymer which contains a disaccharide as repeating unit, wherein the chain comprises 250 to 50.000 of the disaccharide units. Said disaccharide comprises D-glucuronic acid and N-acetyl-D glucosamine being bound via a glycosidic $\beta$-(1,3) linkage. Hyaluronic acid can be present in form of its salts such as alkaline metal or earth alkaline metal salts, in particular in form of the sodium or potassium salt.

Gellan (also referred to as gellan gum) is an anionic polysaccharide obtained via fermentation of carbon hydrates by a bacterium called *Pseudomonas elodea.* Gellan is a polymer containing a tetrasaccharide as repeating unit, wherein the chain comprises 250 to 50.000 of the tetrasaccharide units. Said tetrasaccharide units comprise two residues of D-glucose, one residue of L-rhamnose and a residue of D-glucuronic acid in the following order: D-glucose-D-glucuronic

acid-D-glucose-L-rhamnose. A part of the D-glucose residues can be esterified with acetic acid and/or glycerol acid. Further, the D-glucuronic acid residues can be present in form of a salt, preferably in form of the potassium, sodium, magnesium and calcium salt.

A polypeptide is a chain of amino acids linked together, wherein a single polypeptide chain might make up the entire primary structure of a simple protein. More complex proteins are formed when two or more polypeptides are linked together. Polypeptides can be naturally occurring or obtained via peptide synthesis, for example via the Merrifield Synthesis.

A cellulose based hydrogel is a hydrogel based on cellulose and/or its derivatives. Cellulose is a polysaccharide containing a linear chain of several hundred to many thousands of $\beta(1\rightarrow4)$-linked D-glucose units. Within the present application cellulose derivatives are for example cellulose ether and cellulose ester as well as their salts. Examples of cellulose ethers are hydroxyalkyl cellulose, in particular hydroxy $C_{1-6}$-alkyl cellulose such as hydroxymethyl cellulose, hydroxyethyl cellulose, hydroxypropyl cellulose, hydroxyisopropyl cellulose and hydroxybutyl cellulose, preferably hydroxymethyl cellulose and/or hydroxyethyl cellulose. Examples of cellulose esters are carboxyalkyl cellulose, in particular carboxy $C_{1-6}$-alkyl cellulose such as carboxymethyl cellulose, carboxy ethyl cellulose, carboxypropyl cellulose, carboxybutyl cellulose and/or their salts. Preferred are carboxymethyl cellulose and carboxy ethyl cellulose and their salts, in particular the sodium salt. Further, a mixture of the mentioned compounds can be used. The number average molecular weight of cellulose and/or its derivates is 1.000 g/mol to 250.000 g/mol, preferably 5.000 g/mol to 175.000 g/mol, in particular 10.000 g/mol to 100.000 g/mol, determined with gel permeations chromatography.

The selected hydrogel comprised by the present composition can preferably be based on synthetic polymeric material. Non-limiting examples are polyalkylene oxide-based hydrogel, poly(meth)acrylate-based hydrogel, poly(eth) acrylate-based hydrogel, poly alkyl(meth)acrylate-based hydrogel, poly alkyl(meth)acrylate-based hydrogel, vinyl polymer-based hydrogel, polycaprolactam and polycaprolactone-based hydrogel, polyurethane-based hydrogel, polyurea-based hydrogel and polyurethane-polyurea-copolymer-based hydrogel.

In a preferred embodiment the synthetic polymeric material has a number average weight of 2.500 to 250.000.000 g/mol, preferably 5.000 to 5.000.000 g/mol, in particular 50.000 to 1.000.000 g/mol.

A polyalkylene oxide is a compound which can be represented by the formula $H\text{-}(O\text{-}A)_n\text{-}OH$, wherein A is an alkylene group, preferably a linear alkylene group comprising 2 to 6 carbon atoms, in particular 2 or 3 carbon atoms. Preferred polyalkylene oxides are polyethylene oxide (also referred to as polyethylene glycol), polypropylene oxide (also referred to as polypropylene glycol) and a copolymer from polyethylene oxide and polypropylene oxide.

Poly(meth)acrylate and poly(eth)acrylate are polymers obtained by polymerisation of the corresponding acid, i.e. (meth)acrylic and eth(acrylic) acid, respectively. Polyalkyl(meth)acrylate and polyalkyl(eth)acrylate are alkylesters, preferably alkylesters with 1 to 6 carbon atoms, in particular methyl or ethyl ester of the beforementioned (meth)acrylic and eth(acrylic) acids, respectively.

Vinyl polymer is a polymer derived from compounds containing a vinyl group, wherein the vinyl group can be substituted or unsubstituted, preferably substituted. Substituents can be aromatic groups such as benzene, alkyl groups, preferably $C_1$ to $C_6$ alkyl groups, or other substituents such as halogens, hydroxy and nitrile. Particularly preferred is polyvinyl alcohol.

Polycaprolactam and polycaprolactone are the polymers of the corresponding caprolactam and caprolactone. Caprolactam and caprolactone can be substituted, for example with the substituents as mentioned above, or unsubstituted, preferably unsubstituted.

Polyurea is a polymer represented by the following formula

$$\text{-[-NH-R-NH-(C=O)-NH-R'-NH-(C=O)-]}_n$$

wherein R and R' are aliphatic or aromatic residues.

Polyurethane (PUR and PU) is a polymer composed of organic units joined by urethane (carbamate) links. Polyurethane is represented by the following formula

$$\text{[-(C=O)-NH-R-NH-(C=O)-O-R'-O)-]}_n$$

wherein R and R' are aliphatic or aromatic residues.

In a particularly preferred embodiment of the invention the selected hydrogel comprised by the present composition is a hydrogel based on a polyurethane-polyurea-copolymer. The polyurethane-polyurea-copolymer can be preferably obtained by reacting a mixture comprising a prepolymer having at least two isocyanate end groups, a diamine and a polyhydric alcohol.

It is preferred that the prepolymer having at least two isocyanate end groups has two to four isocyanate end groups, in particular two isocyanate end groups. It is further preferred that the prepolymer having at least two isocyanate end groups is an aliphatic prepolymer having at least two isocyanate end groups. In a particularly preferred embodiment,

the prepolymer having at least two isocyanate end groups is a prepolymer having two isophorone cyanate end groups. The prepolymer having at least two isocyanate end groups can preferably be present in the mixture in an amount of 5 to 20 wt.%, preferably 6 to 18 wt.%, in particular 8 to 16 wt.%, based on the total weight of the mixture.

In particular, the isocyanate-terminated prepolymer is a three-armed copolymer of ethylene oxide and propylene oxide units, each of which has been reacted at the end with a molecule of isophorone diisocyanate. It usually has a content of reactive isocyanate end groups (NCO groups) of 2.5% to 4.0%, preferably 3.0% to 3.4%, particularly preferably 3.2%, and a weight ratio of ethylene oxide units to propylene oxide units of 3 : 1 to 4 : 1 on. Such an isocyanate-terminated prepolymer with aliphatic isocyanate groups is commercially available, for example, as Aquapol® PI-13000-31 (Carpenter; Richmond, USA).

The following figure illustrates the schematic structure of a three-arm branched isocyanate-terminated prepolymer containing polyethylene oxide and polypropylene oxide units (as in Aquapol®). A glycerol molecule forms the centre of the prepolymer. The three "arms" of the prepolymer, each with a terminal isocyanate group, are linked to the hydroxyl groups of the glycerol molecule. The glycerol molecule itself is not shown in the figure. It would be in the right half of the picture where the three "arms" shown schematically as a wavy line meet. The chemical structure of an "arm" is shown in more detail in the left half of the picture.

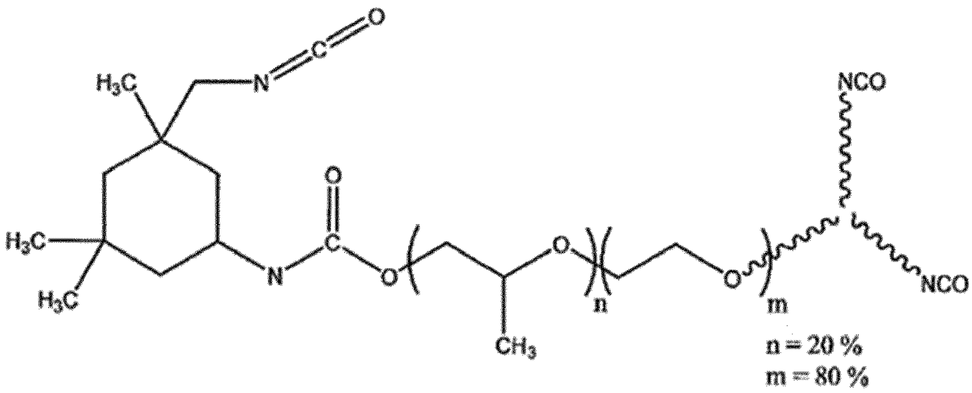

The diamine comprised in the mixture can preferably be an aliphatic diamine. It is more preferred that the diamine is an alkylene oxide-based diamine. An alkyleneoxide-based diamine is an alkylene oxide whose hydroxy end groups are substituted to amine groups. An alkylene oxide-based diamine can also be referred to as polyetheramine. It is preferred that alkylene is alkylene having 1 to 6 carbon atoms, preferably 1 to 4 carbon atoms. Particularly preferred are methylene, ethylene, propylene and mixtures thereof, especially a mixture of ethylene and propylene. Diamine can preferably be present in the mixture in an amount of 5 to 70 wt.%, provided that the ratio of the isocyanate groups (of the prepolymer) to amino groups (of the diamine) is 1.15 to 1.45, preferably 1.20 to 1.40, in particular 1.25 to 1.35, based on the total weight of the mixture.

A polyhydric alcohol preferably comprises diols, triols, tetrols, pentols and hexols and mixtures thereof, more preferably diols, triol, hexols and mixtures thereof. In particular the polyhydric alcohol can be selected from glycols, especially ethylene glycol and propylene glycol, sorbitol and glycerol and mixtures thereof. The polyhydric alcohol can preferably be present in the mixture in an amount of 5 to 50 wt.%, preferably 10 to 45 wt.%, in particular 15 to 40 wt.%, based on the total weight of the mixture. Polyhydric alcohols are excellent moisturizing agents and, thus, provide a nourishing component for the skin surrounding the wound.

Further, the mixture can preferably comprise an inorganic salt. The inorganic salt comprises preferably an inorganic halide, in particular chloride. Further, the inorganic salt is an alkali metal or earth alkali metal salt. Examples are sodium chloride, potassium chloride, magnesium chloride and calcium chloride. In a particularly preferred embodiment, the inorganic salt is sodium chloride. The inorganic salt can preferably be present in the mixture in an amount of 0 to 5 wt.%, more preferably 0.5 to 3 wt.%, in particular about 1 wt.%, based on the total weight of the mixture.

As described above, the polymeric material can be regarded as a matrix or a hydrogel matrix, in particular a dry hydrogel matrix. The presently selected hydrogel matrices can absorb water and thus, subsequently are regarded as specifically selected polymeric material-based hydrogels. The corresponding hydrogels are especially suitable to store water and then to deliver it to the wound to keep the wound humid.

In a preferred embodiment, the hydrogel contains at least 20 wt.%, preferably at least 30 wt.%, more preferably at least 40 wt.%, in particular at least 50 wt.% of water, wherein the hydrogel preferably contains at most 90 wt.%, more preferably at most 80 wt.% of water. Thus, a wound system can be provided that on the one hand is able to provide water to maintain the wound sufficiently humid for a natural wound healing and on the other hand is able to absorb undesired fluid such as exudate from the wound.

In line with the present invention the amount of water contained in the hydrogel should be verified via DIN EN 14079, wherein the amount of water is calculated as follows:

$$W_w = \frac{W_g - W_t}{W_g} \bullet 100\% \qquad (1)$$

wherein

$W_w$ = weight of water in %, based on the total weight of the hydrogel,

$W_g$ = weight of the hydrogel

Wt = weight of the "dry component" of the hydrogel (corresponding to the hydrogel matrix).

In the context of the invention the amount of water should be considered as water that can be theoretically released from the hydrogel. On the contrary, water that is covalently bound should not be considered as belonging to the above-mentioned amount of water.

In a preferred embodiment of the invention the hydrogel is a polyurethane-based hydrogel, more preferably a polyurethane-polyurea-copolymer-based hydrogel. In a preferred embodiment the hydrogel can comprise at least 20 wt.% of water and at least 10 wt.% of polyurethane-polyurea-copolymer. An alternative hydrogel comprises at least 20 wt.% of water and at least 15 wt.% of polyurethane-polyurea-copolymer.

Further, it is preferred that the polymeric syntactic material (hydrogel matrix) is formed from 6 to 60 wt.% of a prepolymer with aliphatic diisocyanate groups, 4 to 40 wt.% polyamine on a polyethylene oxide basis, at least one salt selected from sodium chloride, potassium chloride, magnesium chloride, calcium chloride or mixtures thereof and at least 20 wt.% of water.

Alternatively, it is preferred that the polymeric synthetic material (hydrogel matrix) is formed from 6 to 30 wt.% of a prepolymer with aliphatic diisocyanate end groups, 4 to 20 wt.% polyamine on a polyethylene oxide basis, at least one salt selected from sodium chloride, potassium chloride, magnesium chloride, calcium chloride or mixtures thereof and at least 30 wt.% of water.

Particularly it is preferred that the polymeric synthetic material (hydrogel matrix) is formed from 6 to 20 wt.% of a prepolymer with two isophorone cyanate end groups, 4 to 15 wt.% of diamine on a polyethylene oxide basis, 0.5 to 15 wt.% of a salt selected from sodium chloride, potassium chloride, magnesium chloride, calcium chloride or mixtures thereof and at least 40 wt.% water.

In a preferred embodiment the hydrogel has a thickness of 0.1 to 5.0 mm, more preferably 0.3 to 4.0 mm, in particular 0.5 to 3.0 mm.

The hydrogel comprised by the present infection detection system preferably does not comprise a buffer. Alternatively, the hydrogel comprised by the present infection detection system can comprise a buffer, wherein the buffer substances after being dissolved in demineralized water form a buffer solution having a pH value of 6.0 to 9.0, preferably 6.2 to 8.8, more preferably 6.4 to 8.6 and in particular 6.6 to 8.4.

The term buffer substances as used in the present invention can designate a mixture of chemical substances whose pH value does not change upon addition of an alkaline or acidic compound as much as in an unbuffered system. Generally, the effect of the buffered system is based on the formation of the corresponding weak bases or acid upon addition of oxonium or hydroxide ions. The obtained weak bases or acids show just a minor tendency to dissociate such that they just contribute little to the concentration of oxnonium or hydroxide ions.

The buffer substances are preferably non-toxic, skin-friendly and physiologically harmless compounds. The pH value of the compound arises upon resolving said compound in demineralized water from the balance of the protolysis of the compound. The pH value can be calculated with a good approximation by the Henderson-Hasselbalch equation. In a wound dressing according to the invention the pH value of the corresponding solution, which arises from resolving the puffer substances in demineralized water, is not calculated but measured. For such a measurement 0.1 mol of buffer substance(s) was completely dissolved at 37°C under stirring in one liter of demineralized water. The pH value of the obtained buffer solution can be measured with a commercially available pH meter based on potentiometry such as Labor-Daten-pH-Meter CG841 (Schott Geräte GmbH) with a glass electrode "Flushtrode" (Hamilton Messtechnik GmbH), wherein the pH meter should be calibrated via commercially available calibration solutions prior to use.

Examples of buffer substances as comprised in the wound dressing according to the invention are dihydrogen phosphate/phosphate buffers, bicarbonate/carbonate buffers, hydrogensulfate/sulfate buffers, lactic acid/lactate, glyceric acid/glycerate, gluconate acid/gluconate, acetic acid/acetate, citric acid/citrate, benzoic acid/benzoate, aconitic acid/aconitate, glutaric acid/glutarate, tartaric acid/tartrate, mallow/malate, succinic acid/succinate and glutamic acid/-

glutamate and buffers based on alkaline organic compounds such as primary, secondary or tertiary amines or mixtures thereof.

In a preferred embodiment the buffer is based on an alkaline organic compound, more preferably the buffer comprises a 4-(2-hydroxyethyl)-1-piperazinethane sulfonic acid. 4-(2-hydroxyethyl)-1-piperazinethane sulfonic acid is represented by the following chemical formula:

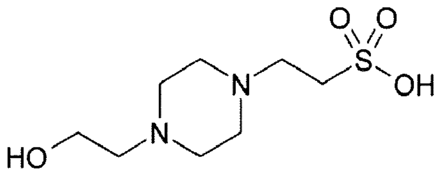

In a further preferred embodiment, the hydrogel contains substances which, when dissolved in demineralized water at 37°C, form a solution exhibiting a pH value of less than 6, amphiphilic substances and/or alcohol in an amount of 0 to 10 wt.%, preferably in an amount of 0.1 to 5 wt.%, in particular 0.2 to 2 wt.%. In a particularly preferred embodiment, the amount of these substances is as small as possible, in particular 0.

Substances which when dissolved in demineralized water at 37°C form a solution exhibiting a pH value of less than 6 can be regarded as acidic substances. The pH of the acidic substances is determined as described above. These substances are for example organic acids such as acetic acid, propionic acid, butyric acid, valeric acid, caproic acid, glycolic acid, lactic acid, pyruvic acid, alpha-ketoglutaric acid, benzoic acid, salicylic acid, acetylsalicylic acid, succinic acid, ascorbic acid, oxalic acid, citric acid, and polyacrylic acid, as well as enolic compounds such as phenol, as well as fatty acids such as caprylic acid, capric acid, lauric acid, myristic acid, palmitic acid, stearic acid, arachidic acid, behenic acid, lignoceric acid, cerotic acid, linoleic acid, myristoleic acid, palmitoleic acid, linolenic acid, arachidonic acid, erucic acid, docosahehaxanoic acid, as well as amino acids, in particular proteinogenic amino acids such as L-alanine, L-arginine, L-asparagine, L-aspartic acid, L-cysteine, L-glutamic acid, L-glutamine, L-glycine, L-histidine, L-isoleucine, L-leucine, L-lysine, L-methionine, L-phenylalanine, L-proline, L-serine, L-threonine, L-tryptophane, L-tyrosine, L-valine, L-selenocysteine, L-pyrrolysine, furthermore their D-enantiomers and non-proteinogenic amino acids.

Amphiphilic compounds, often also referred to as surfactants, are composed of a non-polar and a polar part. The non-polar part can be for example an alkyl chain or an alkyl phenyl group.

The polar part of the amphiphilic compound can be composed of various functional groups being suitable to classify the surfactant into the following four categories: anionic amphiphilic compounds, cationic amphiphilic compounds zwitterionic amphiphilic compounds and non-ionic amphiphilic compounds. Particularly preferred are non-ionic amphiphilic compounds having for example one or a plurality of hydroxy or ether group(s) or combinations thereof.

Examples of non-ionic amphiphilic compounds are fatty alcohols, polyoxyethylene glycol phenyl ethers, polyoxypropylene glycol alkyl ethers, glucoside alkyl ethers, polyoxyethylene glycol sorbitan alkyl ester, sorbitan polyoxyethylene glycerol esters, polyetherramines, polyoxyethylene sorbitol esters, polyoxyethylene sorbitan esters, polyoxyethylene esters, glycerol monoesters, glycerol diesters, polyvinyl or mixtures thereof. Preferred are polyetheramine (also known as Jeffamine) and polyethylene glycol phenyl ether such as polyethylene glycol p-(1,1,3,3-tetramethylbutyl)-phenyl ether (also known as Triton X-100). Alternatively preferred is free Jeffamine. Free Jeffamine can be referred to as Jeffamine in its free form; i.e. not in form of an acidic or basic addition salt nor bonded to another molecule.

Alcohols are organic compounds in which at least one carbon atom is bound to a hydroxyl group as unique functional group. The alcohol can be a polyhydric alcohol, preferably a diol or a triol, i.e. two or three carbon atoms are bound to a hydroxy group, respectively. In a preferred embodiment the alcohol comprises 1 to 12 carbon atoms, preferably 2 to 9carbon atoms, more preferably 3 to 6 carbon atoms. Examples of alcohol are methanol, ethanol, propanol, isopropanol, hexanol, glycol, diethylene glycol, dipropylene glycol, hexane-1,6-diol, sorbitol and glycerol, in particular diethylene glycol, dipropylene glycol and glycerol.

In a preferred embodiment, the hydrogel is one or a mixture of the hydrogel(s) listed in below Table 1:

Table 1: Hydrogels and selected properties.

| | kind | Polymer [%] | NCO, prepolymer [g] | Abs [g/g] | Moisture donation [mg/cm$^2$] | Adhesive force [N] | Elongation at break [N] | Gel point [s] |
|---|---|---|---|---|---|---|---|---|
| Agar-based hydrogel | non-ionic | 12.25 | 2 | 2.45 | 33.07 | 0.1 | 0.96 | 1410 |

(continued)

| | kind | Polymer [%] | NCO, pre-polymer [g] | Abs [g/g] | Moisture donation [mg/cm$^2$] | Adhesive force [N] | Elongation at break [N] | Gel point [s] |
|---|---|---|---|---|---|---|---|---|
| Starch-based hydrogel | anionic | 12.25 | 2 | 2.4 | 25.5 | 0.27 | 0.62 | nd |
| CMC-based hydrogel | anionic | 12.25 | 2 | 6.31 | 41.72 | 0.2 | 0.23 | 1110 |
| Alginate-based hydrogel | anionic | 12.25 | 2 | 2.33 | 33.07 | 0.1 | 0 | 870 |
| Chitosan-based hydrogel | cationic | 12.25 | 2 | 2.66 | 15.07 | 1 | 1.04 | 810 |
| "Abs" corresponds to "absorption"<br>"nd" corresponds to "not-determined" | | | | | | | | |

The infection detection system according to the present invention *inter alia* comprises particles comprising a liposome and a first dye.

In the context of the present application, the term "particles" is understood to mean all particles, regardless of their shape, whose equivalent diameter is in the order of magnitude of 50 nm to 1000 μm (such particles are also generally referred to as microparticles).

Particles in which a substance, for example a dye, is evenly distributed more or less finely in the polymer are also referred to as micromatrices. The particles used are preferably micromatrices or microcapsules. The particles preferably have a diameter of 50 nm to 1000 μm, particularly preferably 5 μm to 300 μm.

A liposome can be regarded as a medium providing a first dye to the wound dressing. The liposome is preferably in form of a particle, wherein said particle comprises, preferably encapsulates, the first dye and wherein said liposome ensures that said first dye is not released from the liposome until in contact with substances such as endotoxins, for example toxins produced by bacteria such as S. aureus and/or P. aeruginosa. It is further preferred that, as long as the first dye is encapsulated completely in an undamaged particle, no coloration of the dye upon excitation with the corresponding excitation wavelength of the dye can be detected outside the particle.

A liposome can be considered as vesicle, preferably a spherical vesicle, that is formed by at least one lipid bilayer. Lipids can be used as structural components in cell membranes, as energy reservoir or signalling molecules. Lipids can be regarded as substances of substantially biological origin wherein at least a part of the lipid is non-polar and thus soluble in non-polar solvents, such as pentane, hexane and benzene. Nevertheless, most of the lipids are amphiphilic, i.e. apart from the beforementioned non-polar, hydrophobic part they comprise a polar hydrophilic part, in particular a hydrophilic end group. Generally, lipids can be categorized in the following groups: triglycerides such as fats and oils, waxes, sphingolipids, lipopolysaccharides, fatty acids, phospholipids and isoprenoids such as steroids and carotenoids.

In a preferred embodiment the lipid bilayer of the liposome contains two amphiphilic lipids such as sphingolipids, fatty acids, phospholipids and isoprenoids such as sterols. Preferred are phospholipids, sterols and 10,12-tricosadinoic acid.

Phospholipids are a group of lipids containing a phosphate group. Due to their chemical structure, phospholipids comprise two groups, namely glycerophospholipids (also referred to as a phosphoglyceride with glycerine as base structure) and sphingomyelins being a phosphor-containing sphingolipid derived from sphingosine. Sterols (also referred to as steroid alcohols) are represented by the following base structure:

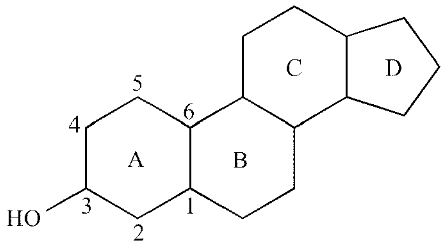

Sterols can for example be produced by some bacteria and the most familiar type is cholesterol, which is vital to cell membrane structure and functions as a precursor to fat-soluble vitamins and steroid hormones. Further examples are phytosterols, such as campesterol, sitosterol and stigmasterol, as well as ergosterol being present in the cell membrane of fungi.

10,12-tricosadinoic acid is a linear carboxylic acid having 23 carbon atoms and two triple bonds at positions 10 and 12.

In a preferred embodiment the liposome comprises

i) at least one of 1,2-dipalmitoyl-sn-glycero-3-phosphocholine (also referred to as DPPC), 1,2-distearyl-sn-glycero-3-phosphocholine, 1,2-dipalmitoyl-sn-glycero-3-phosphoethanolamine (also referred to as DPPE), 1,2-distearyl-sn-glycero-3-phosphoethanolamine and 10,12-tricosadiynoic acid (also referred to as TCDA); and

ii) a sterol.

More preferably the liposome comprises:

i) 1,2-dipalmitoyl-sn-glycero-3-phosphocholine,1,2-dipalmitoyl-sn-glycero-3-phosphoethanolamine and 10,12-tricosadiynoic acid; and

ii) cholesterol.

In a polar solvent a lipid bilayer is preferably formed by two lipids which are orientated such that the corresponding hydrophobic parts of the lipids are directly facing each other and the corresponding hydrophilic parts are at the far ends of the bilayer. An exemplary structure of the lipid bilayer and the liposome are shown in Figures 1 and 2, respectively.

It turned out that the lipid bilayers of the liposome are formed by phospholipids, such as 1,2-dipalmitoyl-sn-glycero-3-phosphocholine and 1,2-dipalmitoyl-sn-glycero-3-phosphoethanolamine, and that 10,12-tricosadiynoic acid and cholesterol enhance the stability of the liposome.

As indicated above, the wound dressing according to the present invention comprises a liposome and a first dye, preferably encapsulated by the liposome.

A dye is a colorant being soluble in water and/or organic solvent. They can be inter alia classified by their origin (natural dyes or synthetic dyes) or by its structure.

In a preferred embodiment the first dye is a fluorescent dye. A fluorescent dye is a fluorescent chemical compound able to re-emit light upon excitation. It is particularly preferred that the fluorescent dye is excited with a wavelength in the UV range, preferably 100 to 380 nm, more preferably 200 to 300 nm, in particular about 250 nm, especially 254 nm. It is further preferred that the re-emitted light has a wavelength in the visible range, preferably from 400 to 750 nm, more preferably from 500 to 600 nm, in particular about 520 nm, especially 517 nm.

Fluorescent dyes are often grouped into classes such as acridine dyes, cyanine dyes, fluorone dyes, oxazine dyes, phenanthridine dyes and rhodamine dyes. Preferred are fluorone dyes.

In a preferred embodiment of the invention the fluorescent dye belongs to the group of xanthenes bearing at least one hydroxy group at the xanthene skeleton. Fluorescent dyes based on a xanthene system bearing at least one hydroxy group at the xanthene skeleton are for example eosins such as eosin B and eosin Y and fluorescein dyes such as 6-carboxyfluorescein (also referred to as carboxyfluorescein), 2,7-dichlorofluorescein and fluorescein.

In a particularly preferred embodiment, the fluorescent dye is fluorescein or carboxyfluorescein, especially carboxyfluorescein.

An exemplary structure of fluorescent dye, encapsulated in high concentrations in the bilipid layer of the carrier, and excited at 254 nm is shown Figure 3. As can be seen from said Figure 3, no fluorescent dye is released from the liposome (undamaged capsules of the lipid bilayer) and, thus, no visible light can be detected. Contrary, in the presence of bacterial toxins the lipid bilayer is damaged, the fluorescent dye can be released from the capsule and thus, the dilution of the florescent dye in the hydrogel matrix takes place, which can be detected as visible light (see Figure 4). Thus, said

particles b) can be considered as a detector for a specific marker, namely the presence of endotoxins which is one indication for an infection. Usually, the presence of bacteria producing endotoxins is the first step of an infection. Thus, the first indication of an infection might be the detection of the first dye, respectively the detection of the visible light thereof.

Further, the infection detection system according to the present invention *inter alia* comprises c) particles comprising a carrier and pH indicator and/or particles comprising a pH-sensitive polymer and a second dye.

In one alternative, the infection detection system according to the present invention *inter alia* comprises particles comprising a carrier and pH indicator. The carrier can be regarded as a medium on which the pH indicator can be applied, or which encapsulates the pH indicator. Is it preferred that the carrier is a polymeric material, preferably a natural or a synthetic polymer.

Not limiting examples for a carrier are natural polymers such as starch, dextran, pectin, alginate, chitosan, hyaluronic acid, gellan, polypeptide and cellulose. With regard to these natural polymers, the same applies as described above. Optionally, the natural polymeric material can be further processed, for example by chemical derivation such as the formation of esters and ethers or pharmaceutically acceptable salts.

Not limiting examples of a carrier are synthetic polymers such polyalkylene oxide-based hydrogel, poly(meth) acrylate-based hydrogel, poly(eth)acrylate-based hydrogel, poly alkyl(meth)acrylate-based hydrogel, poly alkyl(meth) acrylate-based hydrogel, vinyl polymer-based hydrogel, polycaprolactam and polycaprolactone-based hydrogel, polyurethane-based hydrogel, polyurea-based hydrogel and polyurethane-polyurea-copolymer-based hydrogel. With regard to these synthetic polymers, the same applies as described above.

It is preferred that the carrier is a pH-sensitive polymer. In the context of the present invention, a pH-sensitive polymer is understood to mean a substance or a mixture of substances which is made up of a large number of repeating monomer units and which has a first solubility or dissolution rate and at the same temperature and a second pH value that is different from the first pH value, a second solubility or dissolution rate, which depends on the first solubility or the speed of solution is different. This results in different solubilities or dissolution rates of the polymer at different pH values.

In line with this invention, it is advantageous that the solubility or the dissolution rate of the pH-sensitive polymer is lower at an acidic pH than at a neutral or alkaline pH, since an increase in the pH of the wound to be treated often correlates with an inadequate healing process. It is particularly advantageous that the solubility or dissolution rate of the pH-sensitive polymer at a pH of 4 is lower than at a pH of 8.

In particular, it is advantageous that the dissolution rate of the pH-sensitive polymer in an acetic acid acetate buffer (10 mM acetic acid in demineralized water) and at a temperature of 20°C at a pH of 4.0 is less than 0.1 mg/min/g and/or that the dissolution rate of the pH-sensitive polymer in a TRIS-HCl buffer (10 mM each of Tris (tris(hydroxymethyl) aminomethane) and HCl in demineralized water) and at a temperature of 20°C at a pH of 8.0 is more than 1 mg/min/g is the dissolution rate is determined in a one-litre beaker with a diameter of approx. 105 mm, with 10,000 g of polymer in 500 ml of the respective solution being stirred for 10 minutes (magnetic stirrer, 50 rpm). The amount of undissolved polymer after 10 minutes is determined by filtering, drying and weighing the undissolved substance. The amount of polymer that has dissolved during the 10 minutes is determined from this. Particularly suitable here are polymers that are insoluble in aqueous solution at a pH of less than 6.0 and are soluble in an amount of at least 1% (w/w) at a pH of more than 7.0.

Non-limiting examples suitable as pH-sensitive polymer are cellulose acetate phthalate, cellulose acetate trimellitate, hydroxypropyl methylcellulose acetate succinate, carboxymethyl ethyl cellulose, oxidized regenerated cellulose, pol(meth)acrylates, their copolymers and mixtures. Preferred are polymers including copolymers of methacrylic acid with methyl methacrylate, in particular a methacrylic acid/methyl methacrylate poly copolymer, preferably in a ratio of methacrylic acid/methyl methacrylate poly being 1:1 to 1:2.

Such polymers are available, for example, under the name Eudragit® (Evonik, Darmstadt, Germany). Preferred polymer preparations are available under the names Eudragit® S 100, Eudragit® S 12.5, Eudragit FS 30 D, Eudragit® L 100, Eudragit® L 12.5. Mixtures of these polymer preparations can also be used. These polymers have particularly favourable pH-dependent solution properties. Other suitable polymers are copolymers of methacrylic acid and ethyl acrylate, available for example as Eudragit® L100-55.

In line with the present invention, a pH indicator is a halochromic chemical compound, the absorption and/or emission of which may change depending on the pH value. In other words, a change of color may be determined, preferably visually determined, in a specific range of the pH value.

Non-liming examples are thymol blue, methyl orange, litmus, bromothymol blue, cresol red, phenolphthalein, alizarine yellow.

In a preferred embodiment, the pH indicator changes its color at a pH value being from 6 to 9, more preferably from 6.5 to 8.5, in particular about 8. Examples are litmus, which changes color from colorless to blue at a pH value of about 8.3, bromothymol blue, which changes color from green to blue at a pH value of about 7.5, cresol red, which changes color from yellow to violet at a pH value in the range of about 7 to 8.8, and phenolphthalein, which changes color from colorless to violet at a pH value of about 8.2. Preferred are bromothymol blue, cresol red and phenolphthalein, in particular phenolphthalein.

When using the particles c) comprising a carrier and a pH indicator, a certain colour is visible as long as the pH

value of the wound is in the range (about 5.5 to 6.5) at which good wound healing takes place. If the pH value in the wound shifts to higher values, the colour of the indicator shifts to another colour which can be recognized by the emission of visible light when stimulated appropriately.

In another alternative, the infection detection system according to the present invention *inter alia* comprises c) particles comprising a pH-sensitive polymer and a second dye.

As far as the pH-sensitive polymer is concerned, the same applies as described above.

As far as the second dye is concerned, generally the same applies as described above for the first dye. In a preferred embodiment, the second dye is a fluorescent dye. As far as the fluorescent dye is concerned, again generally the same applies as described above, wherein the first dye and the second dye are preferably not the same fluorescent dye such that the detection of the corresponding infection marker can be carried out independently from each other.

When using the particles comprising a pH-sensitive polymer and a second dye, no second dye is released from the particles as long as the particles are intact. If the pH value in the wound shifts, the particles can be dissolved so that the second dye is released. The dilution of the second dye then takes place, which can be recognized by the emission of visible light when stimulated appropriately.

Thus, particles c) can be considered as a detector for a specific marker, namely the presence of an enhanced pH value which is one indication for an infection. As discussed above, an enhanced pH value of a wound is considered to be higher than the pH value enabling a normal healing, i.e. pH value above 6.5. In line with the present application, an enhanced pH value can be about 6.5, about 6.6, about 6.7, about 6.8, about 6.9, about 7.0, about 7.1, about 7.2, about 7.3, about 7.4, about 7.5, about 7.6, about 7.7, about 7.8, about 7.9, about 8.0, about 8.1, about 8.2, about 8.3, about 8.4, about 8.5, about 8.6, about 8.7or about 8.8. The increase of the pH value often occurs after the presence of endotoxins such that such an increase may be regarded as a second step of an infection. Thus, a second indication of an infection might be the change of colour of the pH indicator or the detection of the first dye respectively the detection of the visible light thereof.

The infection detection system according to the present invention *inter alia* comprises d) particles comprising polymer having at least one carboxylic group and a third dye having a phenolic, an aniline and/or anilide structure.

Non-limiting examples of polymers having at least one carboxylic group are natural polymers, such as pectin, alginate, hyaluronic acid, gellan, certain polypeptides, or functionalized celluloses for example carboxyalkyl celluloses such carboxymethyl cellulose, carboxy ethyl cellulose, carboxypropyl cellulose, carboxybutyl cellulose, and synthetic polymers, such as poly(meth)acrylic acid, as well as mixture thereof.

Preferred as polymers having at least one carboxylic group are natural polymers such as pectin, alginate, hyaluronic acid and carboxymethyl cellulose, in particular alginate.

The number average molecular weight of polymer having at least one carboxylic group is 1.000 g/mol to 250.000 g/mol, preferably 5.000 g/mol to 175.000 g/mol, in particular 10.000 g/mol to 100.000 g/mol, determined with gel permeation chromatography.

In a preferred embodiment the synthetic polymeric material has a number average weight of 2.500 to 250.000.000 g/mol, preferably 5.000 to 5.000.000 g/mol, in particular 50.000 to 1.000.000 g/mol.

The third dye has a phenolic, an aniline and/or anilide structure.

Non limiting examples of the third dye are guaiacol (also referred to as 2-hydroxyanisole or 1-hydroxy-2-methoxybenzene), TMB (also referred to as 3,3',5,5'-tetramethylbenzidine), Fast Blue RR, N-(methoxysuccinyl)-Ala-Ala-Pro-Val-p-nitroanilide, N-(methoxysuccinyl)-Ala-Ala-Ala-p-nitroanilide and Peptidoglycan Remazol Brilliant Blue. Preferred are TMB and guaiacol, in particular guaiacol.

In case that HNE (human neutrophil elastase) should be detected, N-(methoxysuccinyl)-Ala-Ala-Pro-Val-p-nitroanilide as third dye is preferred.

In case that CatG (cathepsin G) should be detected, N-(methoxysuccinyl)-Ala-Ala-Ala-p-nitroanilide as third dye is preferred.

In case that lysozyme should be detected, Peptidoglycan Remazol Brilliant Blue as third dye is preferred.

In case that myeloperoxidase (MPO) should be detected, guaiacol, TMB or Fast Blue can be used as third dye, preferably guaiacol and TMB, in particular guaiacol.

In a preferred embodiment the particles comprising polymer having at least one carboxylic group and a third dye having a phenolic, an aniline and/or anilide structure further comprise chitosan. As far as chitosan is concerned, the same applies as described above. It is preferred that chitosan partially or completely encapsulates the corresponding particle. The use of chitosan is considered to enhance the stability of the corresponding particle.

When using d) particles comprising polymer having at least one carboxylic group and a third dye having a phenolic, an aniline and/or anilide structure, no colour from the third dye can be detected as long as the particles are intact. If a host response is carried out and an enzyme associated with said host response became present in the wound, the third dye shifts to a different colour (brownish in case of guaiacol) which can be recognized by visual detection. The enzyme(s) associated with a host response may be selected from the group consisting of lysozyme, cathepsin G, elastase such as HNE (human neutrophil elastase), peptidase such as MMP (metalloproteinase), CRP (c-reactive protein) catalase, lipase, esterase and myeloperoxidase (MPO), in particular myeloperoxidase (MPO).

Thus, particles d) can be considered as a detector for a specific marker, namely the presence of enzymes associated with a host response. In particular, particles d) can be considered as a detector for the presence of MPO (myeloperoxidase) which is an indicator of an infection. The increase of enzymes is considered to be due to the host response which is often regarded as the latest sign of an infection. Thus, the change of colour of the third dye may be regarded as the third step of an infection. Thus, in that case usually all of the particles have indicated an infection such that its presence is reliably confirmed, and the corresponding counter measures should be carried out.

In a preferred embodiment, the first, second and third dyes are different from each other.

In a preferred embodiment of the invention the hydrogel and the particles comprising liposome and first dye are in direct contact with each other, i.e. there are no further layers between them. In one embodiment, the particles comprising liposome and the first dye are preferably applied onto the hydrogel. In a more preferred embodiment, the particles comprising liposome and the first dye are embedded in the hydrogel. Embedded can be referred to as being at least partially surrounded by the hydrogel.

In a preferred embodiment of the invention the hydrogel and the particles comprising a carrier and pH indicator and/or particles comprising a pH-sensitive polymer and a second dye are in direct contact with each other, i.e. there are no further layers between them. In one embodiment, the particles comprising a carrier and pH indicator and/or particles comprising a pH-sensitive polymer and a second dye are preferably applied onto the hydrogel. In a more preferred embodiment, the particles comprising a carrier and pH indicator and/or particles comprising a pH-sensitive polymer and a second dye are embedded in the hydrogel. Embedded can be referred to as being at least partially surrounded by the hydrogel.

In a preferred embodiment of the invention the hydrogel and the particles comprising polymer having at least one carboxylic group and a third dye having a phenolic, an aniline and/or anilide structure are in direct contact with each other, i.e. there are no further layers between them. In one embodiment, the particles comprising polymer having at least one carboxylic group and a third dye having a phenolic, an aniline and/or anilide structure are preferably applied onto the hydrogel. In a more preferred embodiment, the particles comprising polymer having at least one carboxylic group and a third dye having a phenolic, an aniline and/or anilide structure are embedded in the hydrogel. Embedded can be referred to as being at least partially surrounded by the hydrogel.

In a preferred embodiment, the hydrogel can be provided with compartments. Within the present application a compartment is regarded as a three-dimensional shape with distinct boundary surfaces such that the compartment can be clearly distinguished from its surroundings. The compartment can preferably be a three-dimensional shape of any kind, such as a disc-like, pellet-like, spherical, cubic, cuboid, ovoid, droplet-like, teardrop-like, tetrahedron-like and octahedron-like.

The b) the particles comprising liposome and a first dye, c) particles comprising a carrier and pH indicator and/or particles comprising a pH-sensitive polymer and a second dye, and/or d) particles comprising polymer having at least one carboxylic group and a third dye having a phenolic, an aniline and/or anilide structure can be filled or incorporated in the compartment(s).

In a preferred embodiment of the infection detection system according to the present invention, the b) particles comprising liposome and a first dye, c) particles comprising a carrier and pH indicator and/or particles comprising a pH-sensitive polymer and a second dye, and d) particles comprising polymer having at least one carboxylic group and a third dye having a phenolic, an aniline and/or anilide structure are independently placed in different compartments such that just one kind of particles b), c) and d) is placed in any one compartment. This structure enables that each kind of particle can indicate the presence of the corresponding marker (indicator of an infection) without being influenced in any way by the other kinds of particles.

In a preferred embodiment, the infection detection system according to the present invention is in form of a swap kit. That is, the infection detection system, in particular the site of the compartments containing the b) particles comprising liposome and a first dye, c) particles comprising a carrier and pH indicator and/or particles comprising a pH-sensitive polymer and a second dye, and d) particles comprising polymer having at least one carboxylic group and a third dye having a phenolic, an aniline and/or anilide structure is brought in contact by a swap which in turn has been previously brought into contact with wound exudate. Depending on the response of the infection detection system, the wound can be subsequently subjected to an appropriate treatment. For example, in case that just one kind of particles b), c) or d) indicates an infection, it might be decided to await further results before any treatment is initiated, while in case that all kinds of particles b), c) and d) indicate an infection, an appropriate treatment such as the administration of antibiotics seems to be adequate.

A further subject of the present invention is a wound dressing comprising the infection detection system according to the present invention. The infection detection system according to the present invention can indicate the state of the wound and if, for example, there is no indication of an infection, a superfluous change of the wound dressing may be prevented.

The wound dressing according to the present invention may be composed of one or more layers.

For example, the wound dressing according to the present invention may further comprise a wound contact layer. A wound contact layer is a layer being in direct contact with the wound, i.e. there are no further layers between the wound

and the wound contact layer. Wound contact layers are known in the art. To bring the infection detection system into contact with liquids from the wound, such as exudate, the wound contact layer can have a plurality of channels, openings or holes for the passage of liquids; the channels, openings or holes on the first side of the wound contact layer occupying an area of at most 95% of the area of the first side of the wound contact layer. It is further preferably provided that the channels, openings or holes occupy an area of at most 70%, in particular at most 50%, in particular at most 40% and very particularly preferably at most 30% of the area of the first side of the wound contact layer. Very particularly preferably, the wound contact layer has channels, openings or holes which, on the first side of the layer, occupy an area of at least 5% and at most 30% of the area of the first side of the wound contact layer. In particular, the wound dressing has a wound contact layer that has 2 to 8 holes per $cm^2$. It can be provided here that the wound contact layer is a perforated polymer film or a polymer network, in particular a polyurethane film or a polymer network.

The wound dressing may also comprise an absorption/desorption layer and or a backing layer, which are both known in the art.

Another subject of the present invention is the use of a hydrogel comprising compartments containing b) particles comprising liposome and a first dye, compartments containing c) particles comprising a carrier and pH indicator and/or particles comprising a pH-sensitive polymer and a second dye, and compartments containing d) particles comprising polymer having at least one carboxylic group and a third dye having a phenolic, an aniline and/or anilide structure, wherein the first, second and third dyes are preferably different from each other as an infection indicator for wounds.

**Experimental Part:**

**Analytical Methods:**

**Determination of the pH-value of the hydrogel**

To determine the pH value of the prepared hydrogels, a sample with a diameter of 35 mm was punched from the hydrogel and the starting weight of the sample was determined. Together with the fivefold amount of demineralized water, the sample was transferred to a beaker and covered with a glass. The sample was incubated at 23°C (room temperature) for 18 hours and after that period the hydrogel was removed from the sample and the pH value of the remaining liquid was determined in triplicate.

**Determination of the absorption capacity of the hydrogel**

An important factor in modern wound treatment is creating and maintaining a moist wound environment while at the same time wound exudate is adsorbed and encapsulated in the wound dressing. For the characterization of the hydrogels regarding their absorption capacities, the amount of water uptake into the hydrogel is assessed at different points in time.

In this context, samples measuring 30 mm x 30 mm are punched from the gels and their starting weight is determined. Then, the samples are put into beakers and covered with 120 ml demineralized water. The samples are stored at room temperature and taken out of the water at the different testing times (t= 2h, 4h, 6h, 8h, 24h, 30h). Then, they are put on a paper towel for five seconds per side to eliminate excess water before the gels are weighed.

Since the samples have different starting weights, the increase in weights [g] is considered for a better comparability. Each sample is examined in triplicate. The absorption capacity is calculated using the following Formula:

$$W_A\ [g/g] = \frac{M_1 - M_0}{M_0}$$

$W_A$= water absorption capacity [g/g]

$M_0$= starting weight of the sample [g/g]

$M_1$= sample weight at time $t_x$ [g/g]

**Determination of the desorption capacity of the hydrogel**

By maintaining a moist wound, the wound healing process is positively influenced. This is why one of the many requirements a wound dressing has to fulfil is the capacity to release moisture over an extended period of time.

This property is tested by measuring the desorption capacity of the hydrogels. For this test, samples measuring 30 mm x 30 mm are punched from the gels and their starting weights are determined. Then, the samples are stored in a climate cabinet at a temperature of 32°C and a relative humidity of 50% until each of the testing times. At the testing times (t= 2h, 4h, 6h, 8h, 24h, 30h) the samples are taken from the climate cabinet and weighed again to determine weight loss or moisture loss over time. The samples are examined in triplicate. Due to the different starting weights of the samples, the weight ratio of the samples at the different times is calculated using the following Formula for a better comparability.

$$W_D\ [g/g] = \frac{M_1}{M_0}$$

$W_D$= water desorption capacity [g/g]

$M_0$= starting weight of the sample [g/g]

$M_1$= sample weight at time $t_x$ [g/g]

**Preparation of the infection detection system**

**1. Hydrogel**

For the preparation of the hydrogel two separate aqueous liquids A and B were prepared as follows.

Aqueous liquid A:

Sodium chloride (6.24 g; 0.107 mol) was added to sterilized and demineralized water (990 ml) and stirred for five minutes at 23°C to form solution 1.Alternatively, sodium chloride (6.24 g; 0.107 mol) and 2-(4-(2-hydroxyethyl)-1-piperazinyl)-ethane sulfonic acid (1M HEPES in sterilized waster; 10 g) were added to sterilized and demineralized water (990 ml) and stirred for five minutes at 23°C to form buffer solution 1. Solution 1 or buffer solution 1 (767.5 g) and Jeffamine-Mix (50 wt.% Jeffamine and 50 wt.% water; 132.5 g) were mixed at 23°C under stirring for five minutes to form aqueous liquid A (900 g).

Aqueous liquid B:

Aqueous liquid B is an aliphatic isocyanate prepolymer in water (isophorone diisocyanate in water, available under the name Aquapol from Carpenter; 900 g).

The preparation of the hydrogel was conducted by the pilot casting plant B100. Aqueous liquids A and B as prepared above were filled into the casting plant's two cartridges, respectively. To ensure an exact dosage and reproducibility the launch was started not before 12 hours after the filling of the cartridges. By doing this, inaccuracies for example due to bubbles can be prevented. The content of the cartridges was transferred via a pump through different tubes in a mixing chamber, wherein a ratio of aqueous liquid 2 to aqueous liquid 1 was adjusted to 1.5 : 1. In the mixing chamber the two components were stirred by a mixer and the reaction took place. The not completely polymerized hydrogel was released from the mixing chamber and transferred to petri plates, where a complete curing took place.

Properties:

pH value of the obtained hydrogel: 8.45

Adsorption capacity of the obtained hydrogel: see Figure 5

Desorption capacity of the obtained hydrogel: see Figure 6

As can be seen from Figures 5 and 6, most water absorption as well as desorption occurs during the first eight hours, i.e. the present hydrogel is able to keep the wound moist as well as to adsorb wound exudate.

**2.1 Particles comprising liposome and first dye**

For the carrier system the stock solutions with a concentration of 100 mM of 1,2-dipalmitoyl-sn-glycero-3-phosphocholine (DPPC), 1,2-dipalmitoyl-sn-glycero-3-phosphoethanolamine (DPPE), 10, 12-tricosadiynoic acid (TCDA) and cholesterol in chloroform were prepared.

Lipid film:

2120 µl of DPPC stock solution, 80 µl of DPPE stock solution, 1000 µl of TCDA stock solution and 800 µl of cholesterol stock solution were mixed in a vessel under stirring for five minutes. After the removal of chloroform via nitrogen gassing a

lipid film was obtained. To completely remove the chloroform, the obtained product was dried for 60 minutes in a vacuum desiccator at 150 mbar.

Fluorescent dye solution

To carboxyfluorescein (1877 mg), sodium chloride (58 mg) and sodium hydroxide (540 mg) and a sterilely filtrated HEPES buffer solution (0.01 M) in water were added to obtain 100 ml of fluorescent dye solution.

The lipid film as obtained above was dissolved in 20 ml of the fluorescent dye solution under stirring at 70°C for 10 minutes. The lipid mixture was frozen in liquid nitrogen and thawed at 70°C. After performing three freeze-thaw cycles, the mixture was extruded by an extruder (LiposoFast LF-50 by Avestin) at 50°C and a pressure of 20 bar with three subsequent extrusion operations of the mixture. The carboxyfluorescein encapsulated by the carrier was purified via a Sephadex column with HEPES solution (0.01 M) as eluent, wherein particles of the encapsulated fluorescein pass the column faster than not encapsulated fluorescein, to obtain liposome containing (encapsulating) carboxyfluorescein. Liposomes were stored at 4°C for up to 120 hours before UV crosslinking. Liposomes were then transferred into quartz UV cuvettes and crosslinked under UV-C light with a total dose of 90 $Wcm^{-2}$.

**2.2 Particles comprising a carrier and pH indicator**

A homogenous aqueous solution of 20 ml of CaCh (2%) was prepared under stirring by a mixing magnet (liquid A).

Further, 10 ml of an aqueous alginate solution (1.5%) and 0.5 ml of an aqueous phenolphthalein solution (0.1 M) were prepared and combined under stirring (liquid B).

Subsequently, liquid B was added drop by drop at room temperature (25°C) to stirred liquid, wherein particles (beads) comprising alginate and phenolphthalein precipitated, which were isolated.

**2.3 Particles comprising polymer having at least one carboxylic group and a third dye having a phenolic, an aniline and/or anilide structure**

A homogenous aqueous solution of 50 ml of CaCh (2%) was prepared under stirring by a mixing magnet (liquid C).

Further, 20 ml of an aqueous alginate solution (1.5%) and 5 ml of an aqueous guaiacol solution (0.1 M) were prepared and combined under stirring (liquid D).

Subsequently, liquid D was added drop by drop at room temperature (25°C) to stirred liquid C, wherein particles (beads) comprising alginate and guaiacol precipitated (cf. Figure 7), which were isolated.

**3. Infection detection system**

To produce a wound dressing with the indicated properties the liposomes were integrated into the hydrogel as follows.

Still fluid hydrogel mixture of Example 1 was poured into a metal mould having recesses (Step A of Figure 8) After the completion of the polymerization, the hydrogel was separated from the mould, wherein the hydrogel contains recesses (Step B of Figure 8).

Further, a 0.7% solution of hydrogel in HEPES solution (0.01 M) was prepared under stirring at 43°C for 5 minutes. Two parts per volume of this solution were mixed with one part per volume of each of examples 2.1, 2.2 and 2.3 to obtain the filling for the recesses of the hydrogel (Step C of Figure 8). After filling the recesses of the hydrogel with the above-described filling mixture, the filling mixture was cured at 25°C (room temperature).

**4. Detection of infection markers**

**4.1 Fluids**

A simulated wound fluid was prepared by dissolving 9.5 g of MRD (maximum recovery diluent, LOT 00002926) in 1000 ml of sterile water and autoclaving at 121°C. This solution is referred to as control solution.

To a 14.5 ml the simulated wound fluid 0.5 ml Triton was added to obtain a solution simulating the presence of endotoxins. This solution is referred to as endotoxin-simulating solution.

To a 14 ml of the simulated wound fluid 1 ml of NaOH was added to obtain a solution simulating pH change to a higher pH value. This solution is referred to as pH-value-changing solution.

To a 14.5 ml of the simulated wound fluid 0.5 ml $H_2O_2$ was added to obtain a solution simulating presence of

myeloperoxidase (MPO). This solution is referred to as myeloperoxidase-simulating solution.

**4.2 Detection**

An infection detection system based on alginate hydrogel and comprising four compartments (0.5 cm x 0.5 cm) is provided, wherein one compartment is an "empty" control compartment, and the other three compartments each contain one of the particles according to Examples 2.1 to 2.3. The complete system is placed in the above-described MRD (maximum recovery diluent). Subsequently, the complete system is brought into contact with 15 ml of endotoxin-simulating solution, 15 ml of pH-value-changing solution and 15 ml of myeloperoxidase-simulating solution.

The results are shown in Figure 9.

As can been seen from said Figure, when brought into contact with the specific marker, the specific particles suitable to detect a marker comprised in one compartment enable a visual detection of said marker. For example, when incubated with 15 ml of endotoxin-simulating solution, the corresponding fluorescent dye is released from the particles according to Example 2.1 and can be visually detected (cf. upper left corner of the infection detection system in Figure 9). In the present case, due to the presence of several markers, their presence can be visually detected by the colour change of the corresponding dye comprised in a compartment. The presence of several markers as simulated in the present case can be considered as a clear indication of the presence of an infection.

## Claims

**1.** Infection detection system comprising

a) hydrogel
b) particles comprising liposome and a first dye,
c) particles comprising a carrier and pH indicator and/or particles comprising a pH-sensitive polymer and a second dye,
d) particles comprising polymer having at least one carboxylic group and a third dye having a phenolic, an aniline and/or anilide structure.

**2.** Infection detection system according to claim 1, wherein the hydrogel is a polyurethane-based hydrogel.

**3.** Infection detection system according to claim 1 or 2, wherein the liposome comprises

i) at least one of 1,2-dipalmitoyl-sn-glycero-3-phosphocholine, 1,2-distearyl-sn-glycero-3-phosphocholine, 1,2-dipalmitoyl-sn-glycero-3-phospho-ethanolamine, 1,2-distearyl-sn-glycero-3-phosphoethanol-amine and 10,12-tricosadiynoic acid; and
ii) a sterol.

**4.** Infection detection system according to any one of the preceding claims, wherein the first dye is fluorescein.

**5.** Infection detection system according to any one of the preceding claims, wherein the carrier is a natural or synthetic polymer.

**6.** Infection detection system according to any one of the preceding claims, wherein the pH indicator is phenolphthalein.

**7.** Infection detection system according to any one of the preceding claims, wherein the solubility or dissolution rate of the pH-sensitive polymer is lower at a pH of 4 than at a pH of 8.

**8.** Infection detection system according to any one of the preceding claims, wherein the pH-sensitive polymer is a methacrylic acid-co-methyl methylacrylate polymer.

**9.** Infection detection system according to any one of the preceding claims, wherein the second dye is a fluorescent dye.

**10.** Infection detection system according to any one of the preceding claims, wherein the polymer comprising at least one carboxylic group is alginate.

**11.** Infection detection system according to any one of the preceding claims, wherein the third dye having a phenolic

structure, an aniline and/or anilide structure is guaiacol.

**12.** Infection detection system according to any one of the preceding claims, wherein the first, second and third dyes are different from each other.

**13.** Infection detection system according to any one of the preceding claims, wherein the b) particles comprising liposome and a first dye, c) particles comprising a carrier and pH indicator and/or particles comprising a pH-sensitive polymer and a second dye, and d) particles comprising polymer having at least one carboxylic group and a third dye having a phenolic, an aniline and/or anilide structure are independently placed in different compartments such that just one kind of particles b), c) and d) is placed in any one compartment.

**14.** Infection detection system according to any one of claims 1 to 13 in form of a swab kit.

**15.** Wound dressing comprising the infection detection system according to any one of claims 1 to 13.

Figure 1

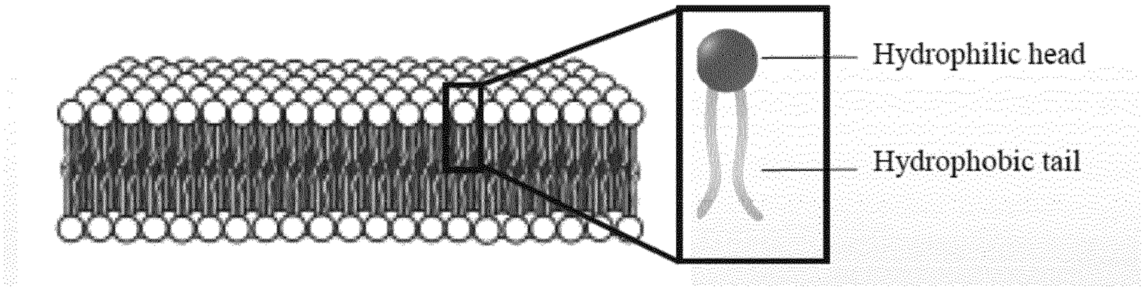


Figure 2

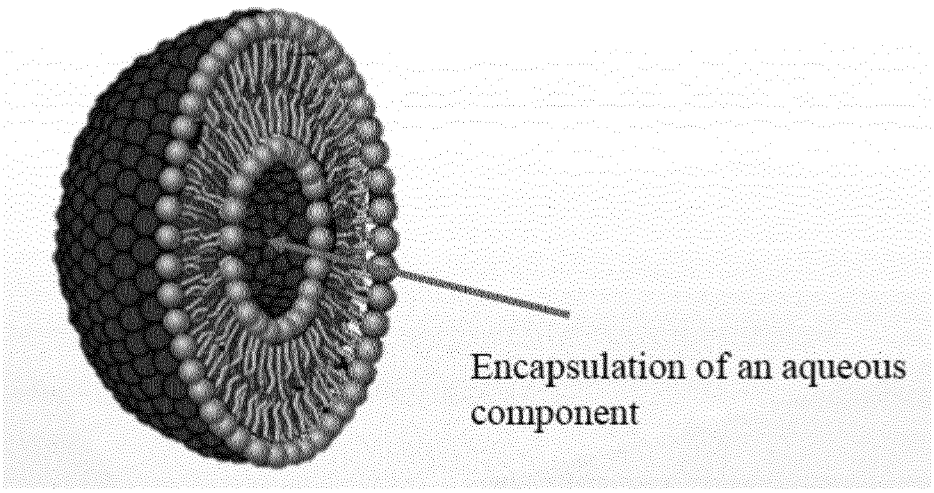

Figure 3

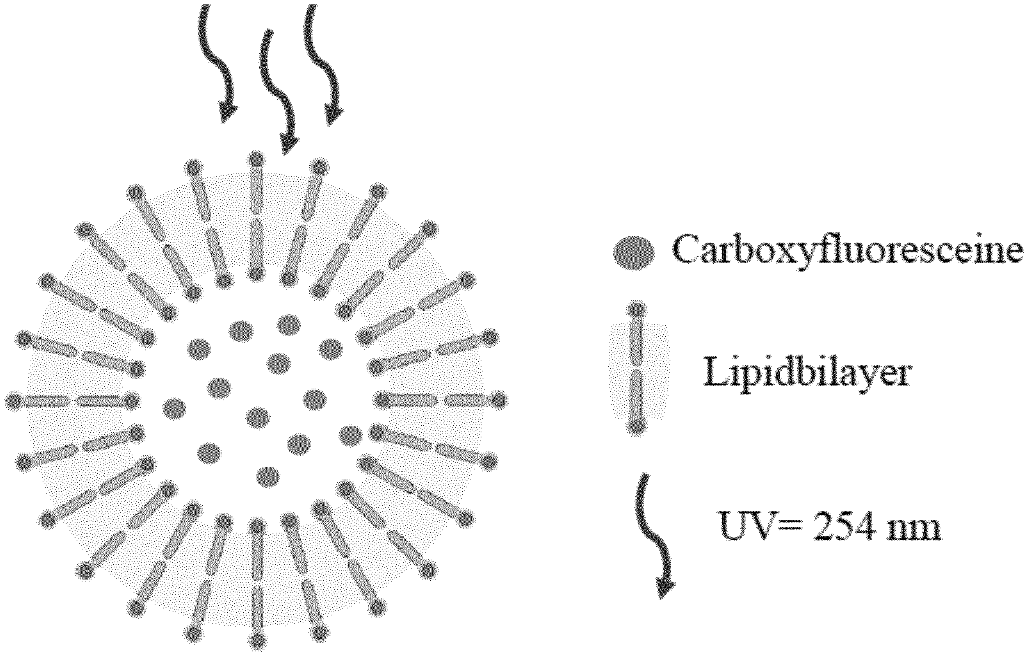


Figure 4

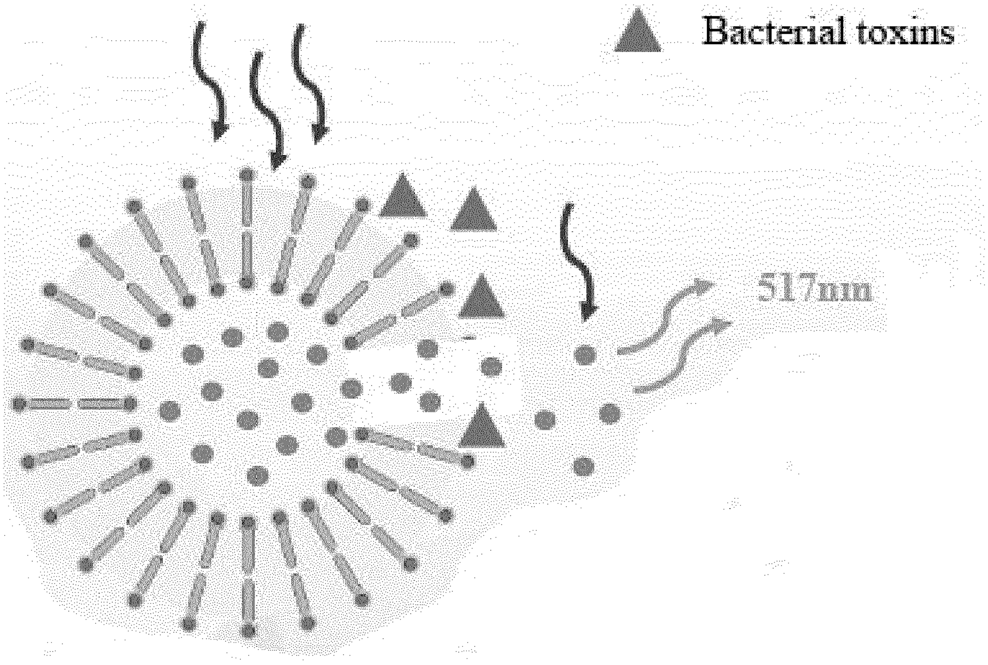

Figure 5

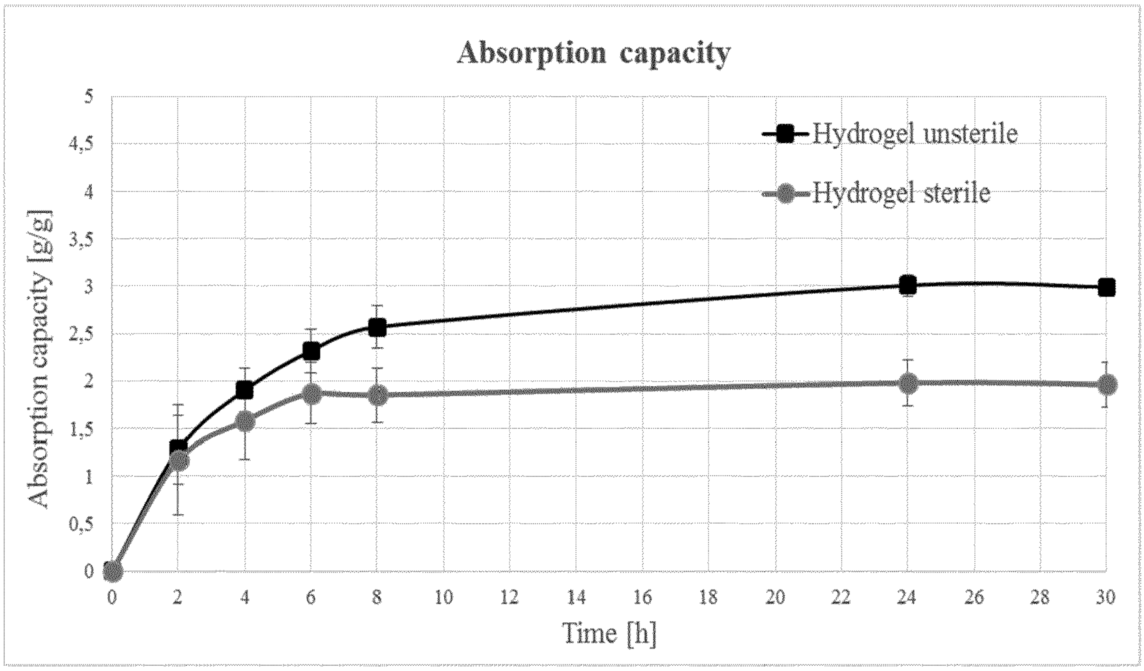


Figure 6

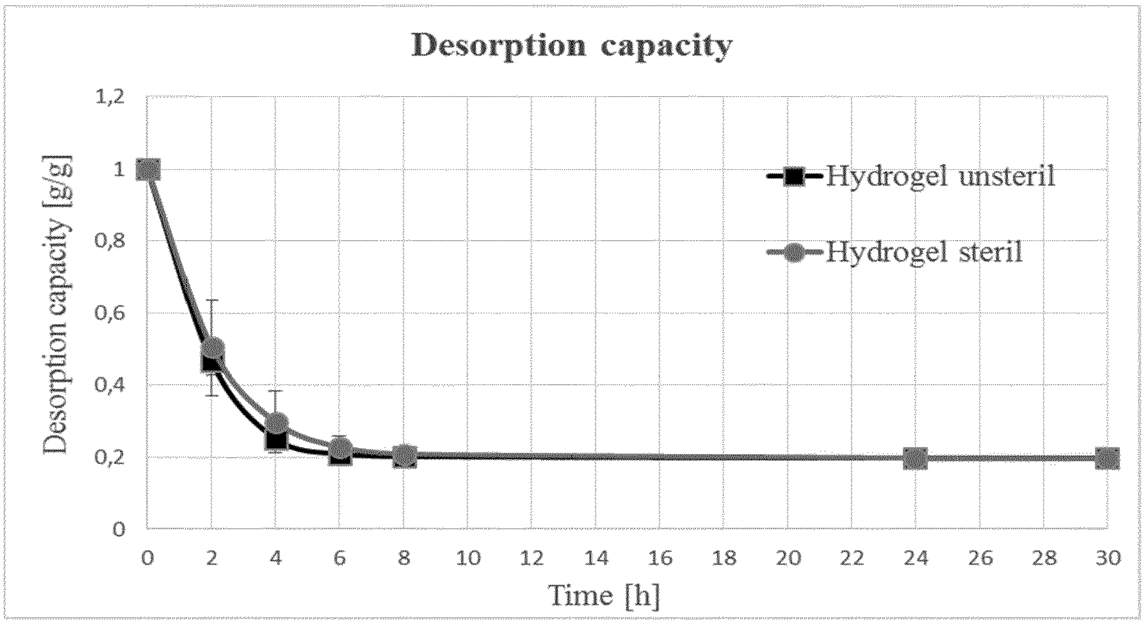

Figure 7

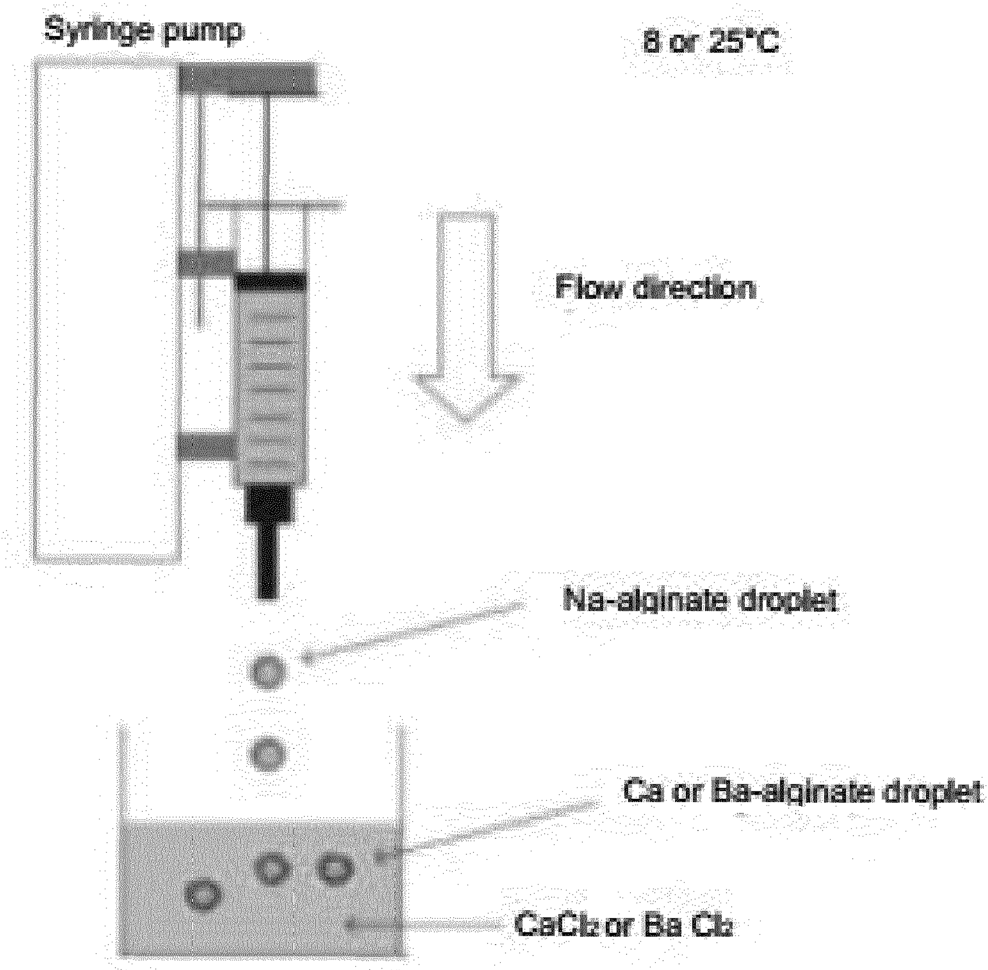


Schematic drawing of alginate bead formation

**Figure 8**

Step A

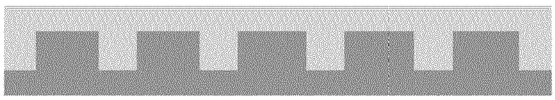

Step B

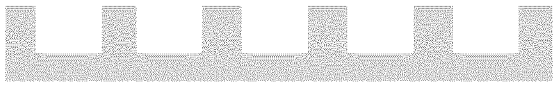

Step C

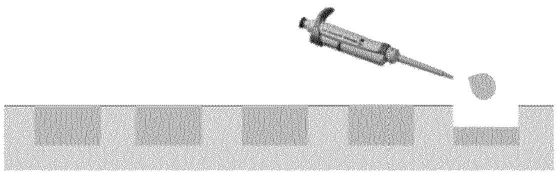

**Figure 9**

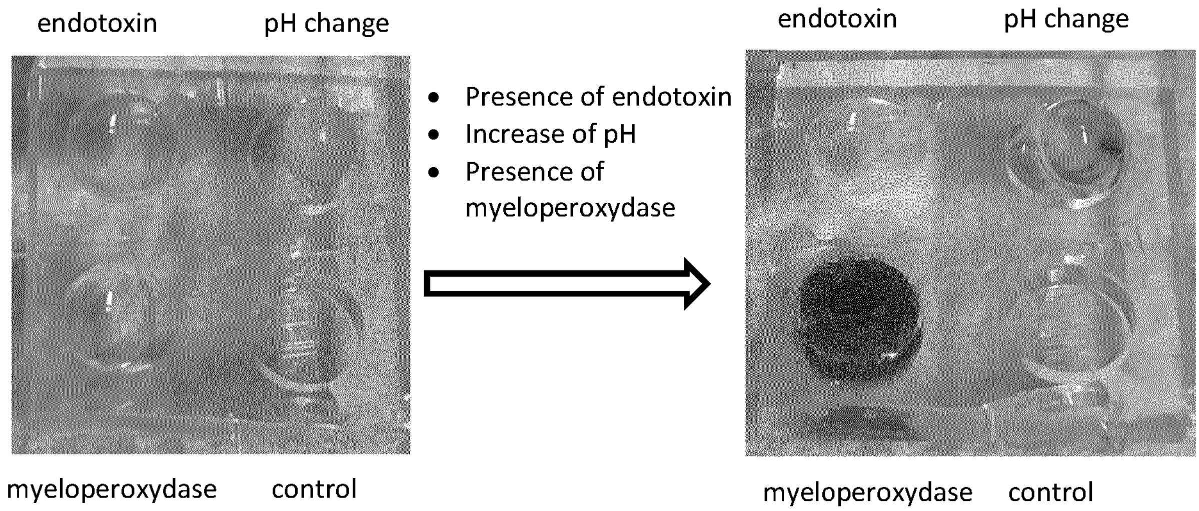

Europäisches Patentamt
European Patent Office
Office européen des brevets

# EUROPEAN SEARCH REPORT

Application Number
EP 23 22 0542

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2021/378565 A1 (KETTEL MARKUS [DE] ET AL) 9 December 2021 (2021-12-09)<br>* paragraphs [0010] - [0027], [0057], [0072] - [0090] *<br>* claims; figures; examples *<br>----- | 1-15<br>1-15 | INV.<br>A61K49/00<br>A61L15/00 |
| Y | WO 2012/074509 A1 (AVERY DENNISON CORP [US]; PATHAK SRIKANT [US]; EDWARDS DAVID N [US]) 7 June 2012 (2012-06-07)<br>* claims; examples *<br>* figures *<br>----- | 1-15 | |
| Y | ZHOU JIN ET AL: "Bacteria-responsive intelligent wound dressing: Simultaneous In situ detection and inhibition of bacterial infection for accelerated wound healing",<br>BIOMATERIALS,<br>vol. 161, 1 April 2018 (2018-04-01), pages 11-23, XP093049458,<br>AMSTERDAM, NL<br>ISSN: 0142-9612, DOI: 10.1016/j.biomaterials.2018.01.024<br>* the whole document *<br>----- | 1-15 | **TECHNICAL FIELDS SEARCHED (IPC)**<br>A61K<br>A61L<br>G01N |
| Y | EP 2 802 312 A1 (UNIV BATH [GB]) 19 November 2014 (2014-11-19)<br>* claims; examples *<br>* abstract *<br>* paragraph [0012] *<br>----- | 1-15 | |

1

The present search report has been drawn up for all claims

| Place of search | Date of completion of the search | Examiner |
|---|---|---|
| The Hague | 4 June 2024 | Ceyte, Mathilde |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 22 0542

This annex lists the patent family members relating to the patent documents cited in the above-mentioned European search report. The members are as contained in the European Patent Office EDP file on
The European Patent Office is in no way liable for these particulars which are merely given for the purpose of information.

04-06-2024

| Patent document cited in search report | Publication date | Patent family member(s) | Publication date |
|---|---|---|---|
| US 2021378565 A1 | 09-12-2021 | CN 113164640 A | 23-07-2021 |
| | | EP 3643328 A1 | 29-04-2020 |
| | | US 2021378565 A1 | 09-12-2021 |
| | | WO 2020084017 A1 | 30-04-2020 |
| WO 2012074509 A1 | 07-06-2012 | EP 2645975 A1 | 09-10-2013 |
| | | JP 2014503252 A | 13-02-2014 |
| | | US 2013261409 A1 | 03-10-2013 |
| | | WO 2012074509 A1 | 07-06-2012 |
| EP 2802312 A1 | 19-11-2014 | EP 2802312 A1 | 19-11-2014 |
| | | US 2015111243 A1 | 23-04-2015 |
| | | WO 2013104876 A1 | 18-07-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012074509 A1


### Non-patent literature cited in the description


- **SHULAVK et al.** Evaluation of pH measurement as a method of wound assessment. *J. Wound Care*, 2007, vol. 16 (7), 291-294